# EUROPEAN PATENT SPECIFICATION

(11) **EP 1 476 562 B1**
(45) Date of publication and mention of the grant of the patent: **06.01.2010**
(21) Application number: 03711011.1
(22) Date of filing: 13.02.2003
(51) Int. Cl.: C12P 21/06, C12N 9/00, C12N 1/20, C12N 15/00, C07H 21/04

(54) **OVER-EXPRESSION OF EXTREMOZYME GENES IN PSEUDOMONADS AND CLOSELY RELATED BACTERIA**
ÜBEREXPRESSION VON EXTREMOZYMGENEN IN PSEUDOMONAS UND NAHE VERWANDTE BAKTERIEN
SUREXPRESSION DES GENES EXTREMOZYME DANS LES BACTERIES PSEUDOMONALES ET BACTERIES ETROITEMENT LIEES

(30) Priority: 13.02.2002 WO PCT/US02/04294
(43) Date of publication of application: 17.11.2004
(73) Proprietor: DOW GLOBAL TECHNOLOGIES INC., Midland MI 48674 (US)
(72) Inventor: CHEW, Lawrence, C., San Diego, CA 92130-4802 (US); TALBOT, Henry, W., San Diego, CA 92130 (US); LEE, Stacey, L., San Diego, CA 92129 (US)
(74) Representative: Raynor, John
(86) International application number: PCT/US2003/004288
(87) International publication number: WO 2003/068926

(56) References cited:
- US-A- 5 840 554
- US-B1- 6 329 172
- LINDEN A ET AL: "Single-step purification of a recombinant thermostable alpha-amylase after solubilization of the enzyme from insoluble aggregates" JOURNAL OF CHROMATOGRAPHY. BIOMEDICAL APPLICATIONS, ELSEVIER, AMSTERDAM, NL, vol. 737, no. 1-2, January 2000 (2000-01), pages 253-259, XP004184274 ISSN: 0378-4347
- LEVEQUE EMMANUEL ET AL: "Thermophilic archaeal amylolytic enzymes" ENZYME AND MICROBIAL TECHNOLOGY, vol. 26, no. 1, January 2000 (2000-01), pages 3-14, XP009055376 ISSN: 0141-0229
- DEMIRJIAN D C ET AL: "ENZYMES FROM EXTREMOPHILES" CURRENT OPINION IN CHEMICAL BIOLOGY, CURRENT BIOLOGY LTD, LONDON, GB, vol. 5, 2001, pages 144-151, XP002901818 ISSN: 1367-5931
- PIRE C ET AL: "Heterologous overexpression of glucose dehydrogenase from the halophilic archaeon Haloferax mediterranei, an enzyme of the medium chain dehydrogenase/reductase family" FEMS MICROBIOLOGY LETTERS 25 JUN 2001 NETHERLANDS, vol. 200, no. 2, 25 June 2001 (2001-06-25), pages 221-227, XP009055378 ISSN: 0378-1097
- FUERSTE J P ET AL: "MOLECULAR CLONING OF THE PLASMID RP-4 PRIMASE REGION IN A MULTI-HOST-RANGE TAC-P EXPRESSION VECTOR" GENE, ELSEVIER BIOMEDICAL PRESS. AMSTERDAM, NL, vol. 48, no. 1, 1986, pages 119-132, XP002321689 ISSN: 0378-1119
- LABES M. ET AL.: 'A new family of RSF1010-derived expression and lac-fusion broad-host-range vectors for gram-negative bacteria' GENE vol. 89, no. 1, May 1990, pages 37 - 46, XP002072525
- SMIDT M. ET AL.: 'Temperature-induced production of recombinant human insulin in high-cell density cultures of recombinant E. coli' J. BIOTECHNOL. vol. 68, no. 1, March 1999, pages 71 - 83, XP004157320
- HASENWINKLE D. ET AL.: 'Very high-level production and export in E. coli of a cellulose binding domain for use in a generic secretion-affinity fusion system' BIOTECHNOL. BIOENG. vol. 55, no. 6, September 1997, pages 854 - 863, XP002967518
- WEYDEMANN U. ET AL.: 'High-level secretion of hirudin by hansenula polymorpha: authentic processing of three different preprohirudines' APPL. MICROBIOL. BIOTECHNOL. vol. 44, no. 3-4, May 1995, pages 377 - 385, XP000914759
- AHN J H. ET AL: 'Homologous Expression of the Lipase and ABC Transporter Gene Cluster, tliDEFA, Enhances Lipase Secretion in Pseudomonas' APPLIED AND ENVIRONMENTAL MICROBIOLOGY vol. 67, no. 12, December 2001, WASHINGTON,DC, US, pages 5506 - 5511, XP002988919

## Description

### BACKGROUND

Enzymes have long found use as biocatalysts in industrial and household processes and, more recently, in medical applications. For example, enzymes are commonly employed in traditional industrial biotechnological processes such as the catalytic liquefaction of corn starch (*e*.*g*., by amylase enzymes), in household processes such as catalytic stain removal (*e*.*g*., by subtilisins and other protease enzymes), and in medical applications such as catalytic thrombolysis for the *in vivo* dissolution of clots (*e*.*g*., by urokinase enzymes). It is widely recognized that enzymes having increased stability under the conditions present in the intended use, a feature typically described in terms of the half-life of the enzyme's activity under such conditions, have greater desirability than those with lesser stability. It is also widely recognized as desirable for the enzyme to exhibit a maximal degree of catalytic activity under the conditions of use, a feature referred to as the enzyme's "optima" (stated in the plural to reflect that the maximum possible level(s) of an enzyme's catalytic activity can vary with different environmental parameters, *e*.*g*., temperature, salinity, pH, etc.). This means that it is most desirable for an enzyme to exhibit both high stability and catalytic optima under the conditions of the intended use.

Many intended uses for enzymes have been proposed wherein the environmental conditions include high or low temperature, high or low pH, high salinity, and other conditions that deviate substantially from the environmental parameters supporting more common living things; among such "more common" biotic conditions are, *e.g*., temperatures of about 20-60°C, pH of about 6.0-7.5, and salinity below about 3.5% (w/v). In order to attempt to fulfill these proposed uses, "extremozymes" have been suggested. Extremozymes are generally considered to be enzymes having significant catalytic activities under extreme environmental conditions, and typically often exhibiting high stability to and catalytic optima under such extreme conditions.

Examples of proposed uses in which extremozymes could offer particular advantages include, *e*.*g*., those listed in Table 2 of M.W.W. Adams & R.M. Kelly, Finding and Using Hyperthermophilic Enzymes, TIBTECH 16:329-332 (1998). Such proposed applications have contemplated the use of extremozymes in:
1. Molecular biology, *e.g*.: employing hyperthermophilic DNA polymerases in the Polymerase Chain Reaction (PCR); use of extremophilic DNA ligases in genetic engineering; extremophilic proteases for use in research;
2. Starch hydrolysis and processing, *e*.*g*.: using alpha-amylases, beta-amylases, glucoamylases, alpha-glucosidases, pullulanases, amylopullulanase, cyclomaltodextrin glucanotransferases, glucose isomerases, and xylose isomerases to produce such products as oligosaccharides, maltose, glucose syrups, high fructose syrups;
3. Chemical synthesis, *e*.*g*.: ethanol production; production of aspartame by thermolysin; production of chiral intermediates for synthesis of pharmaceutical active ingredients; use of other proteases, lipases, and glycosidases having high stability at high temperatures or in organic solvents;
4. Cellulose and gum degradation and processing, *e*.*g*.: paper and pulp bleaching by xylanases; cellobiohydrolases, beta-glucosidases and beta-glucanases for cellulose hydrolysis; thermostable cellulases and glucanases for degradation of biological gums used in oil recovery;
5. Food and feed processing, *e*.*g*.: pectinases, cellulases, and chitinases; galactosidases for lactose hydrolysis; and phytases for dephosphorylation of phytate in animal feed during high temperature processing;
6. Medical treatments and diagnostic devices and kits, *e*.*g*.: peroxidases, phosphatases, oxidases, carboxylases, and dehydrogenases;
7. Detergents and household products, *e*.*g*.: thermophilic proteases, alkalophilic proteases; and, alkaline amylases; and
8. Other industrial applications, *e*.*g*.: biomining and bio-leaching of minerals, bioremediation, remediation of radioactive wastes, antioxidation systems.

The main recognized source for extremozymes is the diverse group of organisms known as extremophiles. Extremophiles are organisms that have been discovered to thrive under extreme environmental conditions, *e*.*g*., in or near deep sea hydrothermal vents, hot springs, high-salinity lakes, exposed desert surfaces, glaciers and ice packs. Members of this group of organisms include representatives from within each of the following categories, *e*.*g*.: prokaryotes including archaea and bacteria, and eukaryotes including fungi and yeasts, lichens, protists and protozoa, algae and mosses, tardigrades and fish. Because organisms of this group naturally thrive under environmental extremes, they are viewed as a source of naturally occurring extremozymes. Accordingly, a number of extremozymes from extremophiles have been isolated and tested, and found to have the desired advantageous properties of high stability and catalytic optima under proposed, extreme conditions of use. However, while the industry has anxiously awaited the expected widespread commercialization of extremozymes, this has not been forthcoming.

The problem is that extremophiles have been found either impossible to culture, or at least too difficult to culture on a commercially significant enough scale to permit cost-effective isolation of extremozymes in sufficient quantity for marketing purposes. As a result, genetic engineering has been tried wherein extremozyme genes, isolated from extremophiles, have been transformed into and expressed in common expression host organisms. Chief among these expression host organisms are *E. coli* and *Bacillus subtilis*. Yet, these expression hosts, which have been found so reliable in producing commercial quantities of non-extremozyme proteins, have so far been unreliable at producing, or unable to produce, commercial quantities of extzemozymes. Thus, at best, in spite of the wealth of potential applications for extremozymes, their use has been limited to specialized, small-scale applications such as thermostable DNA polymerases for use in research; significant industrial scale use has not yet been achieved because of the lack of a commercially viable, industrial scale extremozyme expression system.

Many examples of such attempts at expression of heterologous extremozyme genes have been reported in *E. coli* hosts, and occasionally in *Bacillus* hosts, and the expression levels are typically poor, *i.e*. less than 5% total cell protein. Representative examples include, *e*.*g*.: G. Dong et al., in Appl. Envir. Microbiol. 63(9):3569-3576 (Sep 1997) (*Pyrococcus furiosus* amylopullulanase expressed in *E. coli* at 10-28mg/L, *i.e*. about 1.4% total cell protein (tcp)); E. Leveque et al., in FEMS Microbiol. Lett. 186(1):67-71 (May 1, 2000) (*Thermococcus hydrothermalis* alpha-amylase expressed in *E. coli* at less than 5% tcp, as estimated from SDS-PAGE); A. Linden et al., in J. Chromatog. B Biomed. Sci. Appl. 737(1-2):253-9 (Jan 14, 2000) (*Pyrococcus woesei* alpha-amylase expressed in *E. coli* at 0.4% tcp, as calculated from data presented therein); and C Pire et al., in FEMS Microbiol. Lett. 200(2):221-27 (Jun 25, 2001) (25-40 mg/L yield of a halophilic glucose dehydrogenase expressed in *E. coli*).

Two exceptions to the rule of poor expression of extremozymes are reported, both for hyperthermophilic dehydrogenases expressed in *E. coli,* at levels of 50% tcp and 15% tcp, respectively. See, H. Connaris et al., in Biotech. Bioeng. 64(1):38-45 (Jul 5, 1999) (*Haloferax volcanii* dihydrolipoamide dehydrogenase expressed in *E. coli* at 50% tcp); and J. Diruggiero & F.T. Robb, in Appl. Environ. Microbiol, 61(1):159-164 (Jan 1995) (*Pyrococcus furiosus* glutamate dehydrogenase expressed in *E. coli* at 15% tep). However, even these examples fail to provide a commercially viable, industrial scale extremozyme expression system for the following reasons.

First, the *E. coli* host cells used in the expression systems reported by Connaris and Diruggiero grow on a rich medium, which can support a maximum cell density of about 2 g/L (maximum biomass accumulation stated in terms of dry cell weight). At such a low cell density, even an expression level of 50% tcp (total cell protein), results in a yield far too low for industrial scale production. For example, with a maximum biomass of 2 g/L, the total cell protein content is approximately 1 g/L; thus, at a 50% tcp expression level, only about 0.5 g/L of the extremozyme would be expressed. An expression system providing a total productivity of only about 0.5 g/L extremozyme is far too low to be considered capable of industrial scale production. This is especially highlighted when considered in light of the bulk quantities of extremozymes required to enable market supply for the majority of proposed industrial processing and household product uses (most of which are premised on large-scale, mass production).

Second, the largest scale of fermentation reported by either of the Connaris and Diruggiero references is a one-liter (1 L) fermentation, which is far too low to be considered "industrial scale" fermentation. Generally, the lowest limit for any cognizable industrial scale fermentation is about 10 L, though for most purposes this is still considered a small "seed-scale" fermentor. However, some, small-scale commercial uses can be provided by 5 L or 10 L fermentation if the total productivity of the expression system is sufficiently high. Common "seed-scale" fermentors also include 20 L and 40 L fermentors; common "pilot-scale" fermentors can range from about 50 L to 200 L, 250 L, and even 500 L in volume. Typical industrial scale productions are done in fermentors having a volume of 1,000 L and above; even 10,000 L and 50,000, L fermentors are not uncommon.

Thus, scaling up a 1 L fermentation-scale expression system to industrial scale fermentation is not a trivial matter. Scaling it up in such as way as to provide industrial scale enzyme production is typically quite a challenge, and especially so when starting with a low-productivity expression system such as reported in the Connaris and Diruggiero references. Nor do these references provide any suggestion or guidance as to how to attempt or accomplish such a scale-up with the expression systems they describe.

Third, the use of rich media, *e*.*g*., LB medium and others, requires expensive additives such as peptones and yeast extracts, a fact that makes industrial scale production significantly cost disadvantaged. In fact, for most proposed uses in which extremozymes could replace existing industrial enzymes, this cost disadvantage would make it too expensive to supply extremozymes to the market for industrial use.

Hence, the biotechnology industry continues to lack a commercially viable, industrial scale extremozyme expression system.

Ahn J H, et al ("Homologous Expression of the Lipase and ABC Transporter Gene Cluster, tliDEFA, Enhances Lipase Secretion in Pseudomonas" Applied and Environmetal Microbiology, vol. 67, no. 12, December 2001, pg 5506-5511) describes the expression in *P.fluorescens* and other *Pseudomonas* species of lipase *tliA*, which is a lipase naturally found in *P.fluorescens*. It is indicated, cross-reference being made to an earlier document (Miller, J.H., 1972, "Experiments in molecular genetics"), that *tliA* exhibits optimal activity at 45°C and pH 8.5. The expression of the lipase *tliA* in *P.fluorescens* and other *Pseudomonas* species is also described in US-B-6,329,172.

### SUMMARY OF THE INVENTION

According to a first aspect, the present invention provides a recombinant cell containing an expression vector comprising a nucleic acid sequence encoding an exogenous extremozyme, wherein the recombinant cell is a Pseudomonad, and wherein the extremozyme is a hydrolase of class IUBMB EC 3 and exhibits an optimum of at least one catalytic property under at least one extremophilic condition selected from: hyperthermophilic conditions of 70-130+°C; psychrophilic conditions of -2-20°C; halophilic conditions of 2-5M salt; acidophilic conditions of a pH of ≤ 4.5; alkalophilic conditions of a pH ≥ 9; piezophilic conditions of 10-80+ MPa; and xerophilic conditions of a_{w} < 0.85.

In a preferred embodiment, the host cell is *Pseudomonas fluorescens*.

The extremozyme may, for example, be a hyperthermophilic, psychrophilic, acidophilic, alkalophilic, or halophilic extremozyme.

According to a second aspect, the present invention provides a process for producing a recombinant cell according to the first aspect, the process comprising:
a) providing a host cell selected from a Pseudomonad; and
b) transforming the host cell with an expression vector comprising a nucleic acid including a coding sequence for an exogenous extremozyme;
wherein the extremozyme is a hydrolase of class IUBMB EC 3 and exhibits an optimum of at least one catalytic property under at least one extremophilic condition selected from: hyperthermophilic conditions of 70-130+°C; psychrophilic conditions of -2-20°C; halophilic conditions of 2-5M salt; acidophilic conditions of a pH of ≤ 4.5; alkalophilic conditions of a pH ≥ 9; piezophilic conditions of 10-80+ MPa; and xerophilic conditions of a_{w} < 0.85.

According to a third aspect, the present invention provides a process for producing an extremozyme, the process comprising growing a recombinant cell according to the first aspect under conditions permitting expression of the extremozyme, wherein the extremozyme is expressed at an expression level of at least 5% total cell protein and is isolated.

In preferred embodiments, the cell is grown to a cell density of at least 40 grams per liter, is grown in a fermentation scale of at least 10 liters, and/or is grown in a mineral salts medium.

In preferred embodiments, the extremozyme is purified and/or undergoes a refolding step.

### BRIEF DESCRIPTION OF DRAWINGS

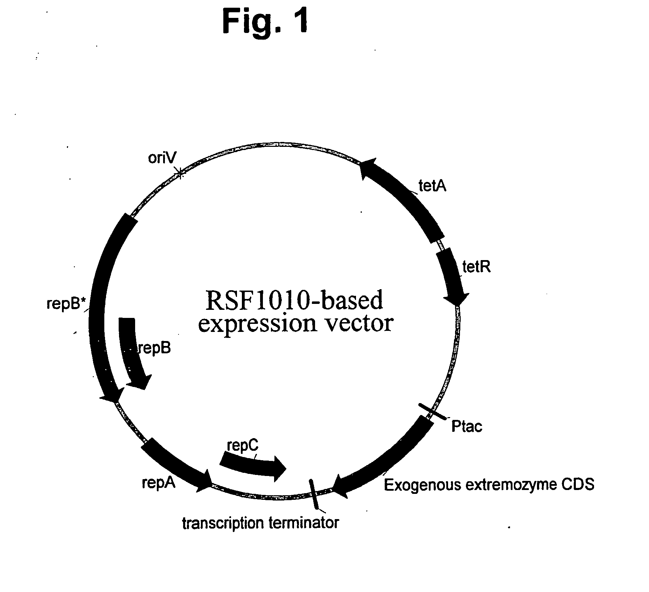

Figure 1 presents a plasmid map of an RSF1010-based expression vector useful in expressing extremozyme genes according to the present invention.

### DETAILED DESCRIPTION OF PREFERRED EMBODIMENTS

The present invention provides a commercial scale production system for extremozymes in which Pseudomonads are used as host cells to over-express the extremozymes.

*Pseudomonas* spp. have previously been used as expression systems. See, *e.g.,* US Patent No. 5,055,294 to Gikoy and US Patent No. 5,128,130 to Gilroy et al.; US Patent No. 5,281,532 to Rammler et al.; US Patent Nos. 5,527,883 and 5,840,554 to Thompson et al.; US Patent Nos. 4,695,455 and 4,861,595 to Barnes et al.; US Patent No. 4,755,465 to Gray et al.; and US Patent No. 5,169,760 to Wilcox. However, in none of these references has it been suggested that Pseudomonads and closely related bacteria would be particularly advantageous at over-expressing extremozymes in commercial quantities, as defined by the present invention.

### GLOSSARY

### A and An

As used herein and in the appended claims, the singular forms "a", "an", and "the" include both singular and plural referents unless the context clearly dictates otherwise. Thus, for example, reference to "a host cell" literally defines both those embodiments employing only a single host cell and those employing a plurality of such host cells.

### In and On

As used herein in regard to growing organisms by use of a growth medium, the organisms may be said to be grown "in" or "on" the medium. In the expression systems of the present invention, the medium is a liquid medium. Thus, in this context, the terms "in" and "on" are used synonymously with one another to indicate growth of the host cells in contact with the medium and generally within the bulk of the medium, though some incidental cell growth at, in, or upon the surface of the medium is also contemplated.

### Comprising

As used herein, the term "comprising" means that the subject contains the elements enumerated following the term "comprising" as well as any other elements not so enumerated. In this, the term "comprising" is to be construed as abroad and open-ended term; thus, a claim to a subject "comprising" enumerated elements is to be construed inclusively, *i.e*. construed as not limited to the enumerated elements. Therefore, the term "comprising" can be considered synonymous with terms such as, *e.g*., "having," "containing," or "including."

The invention, as described herein, is spoken of using the terms "comprising" and "**characterized in that**." However, words and phrases having narrower meanings than these are also useful as substitutes for these open-ended terms in describing, defining, or claiming the invention more narrowly. For example, as used herein, the phrase "consisting of" means that the subject contains the enumerated elements and no other elements. In this, the phrase "consisting of" is to be construed as a narrow and closed-ended term. Therefore, the term "consisting of" can be considered synonymous with, *e*.*g*.: "containing only" or "having solely".

### Depositories

ACAM - Australian Collection of Antarctic Microorganisms, Cooperative Research Centre for Antarctic And Southern Ocean Environment, University of Tasmania, GPO Box 252C, Hobart, Tasmania 7001, Australia.

ATCC - American Type Culture Collection, 10801 University Boulevard, Manassas, VA 20110-2209, U.S.A.

NCIMB - National Collection of Industrial and Marine Bacteria, National Collections of Industrial, Food and Marine Bacteria, 23 Machar Drive, Aberdeen, AB24 3RY, Scotland. UQM - Culture Collection, Department of Microbiology, University of Queensland, St. Lucia, Queensland 4067, Australia.

### General Materials & Methods

Unless otherwise noted, standard techniques, vectors, control sequence elements, and other expression system elements known in the field of molecular biology are used for nucleic acid manipulation, transformation, and expression. Such standard techniques, vectors, and elements can be found, for example, in: Ausubel et al. (eds.), Current Protocols in Molecular Biology (1995) (John Wiley & Sons); Sambrook, Fritsch, & Maniatis (eds.), Molecular Cloning (1989) (Cold Spring Harbor Laboratory Press, NY); Berger & Kimmel, Methods in Enzymology 152: Guide to Molecular Cloning Techniques (1987) (Academic Press); and Bukhari et al. (eds.), DNA Insertion Elements, Plasmids and Episomes (1977) (Cold Spring Harbor Laboratory Press, NY).

X-gal means 5-bromo-4-chloro-3-indolyl-beta-D-galactoside

IPTG means Isopmpylthio-beta-D-galactoside

ORF means Open reading frame.

tcp and %tcp

As used herein, the term "tcp" means "total cell protein" and is a measure of the approximate mass of expressed cellular protein per liter of culture. As used herein, the term "%tcp" means "percent total cell protein" and is a measure of the fraction of total cell protein that represents the relative amount of a given protein expressed by the cell.

### Exogenous and Heterologous

The term "exogenous" means "from a source external to" a given cell or molecule. The term "heterologous" means "from a source different from" a given cell or molecule. In the present application, as is common use in the art, these two terms are used interchangeably, as synonyms. Both of these terms are used herein to indicate that a given object is foreign to the cell or molecule, *i*.*e*. not found in nature in the cell or not found in nature with or connected to the molecule.

### Extremophilic

Extremophilic is defined as any condition falling within the parameters listed in Table 1.

| Table 1. Parameters Defining "Extremophilic" | |
|---|---|
| Extremophilic Condition | Approximate Definition |
| Hyperthermophilic | 70-130+°C |
| Psychrophilic | -2-20°C |
| Halophilic | 2-5M salt |
| Acidophilic | pH≤4.5 |
| Alkalophilic | pH≥9 |
| Piezophilic | 10-80+ MPa |
| Xekophilic* | a_{W} < 0.85 |

| | |
|---|---|
| * - Xerophilic is defined by a dimensionless quantity known as "water potential": a_{W} = [(Vapor Pressure of Water in Liquid Solution)/(Vapor Pressure of Pure Water)], wherein "Liquid Solution" indicates any aqueous medium or aqueous environment, whether intracellular or extracellular. | |

Extremophiles are thus defined as those organisms that readily survive or thrive under extracellular environmental conditions falling within these listed parameters. Extremophilic enzymes, or extremozymes, are likewise defined with reference to the conditions defined in Table 1, and these may be either intracellular or extracellular conditions.

In some cases, chemophilic (*e*.*g*., metalophilic) and radiophilic conditions are also recognized in the art as classes of extremophilic conditions, although these depend on the type of chemical (*e*.*g*., a specific metal or a organic compound) and the type of radiation, and thus no uniform definition is included in the present definition of "extremophilic."

### Enzymes

As used herein, the term "enzymes" includes:
1. Oxidoreductases (IUBMB EC 1: including, *e*.*g*., monooxygenases, cytochromes, dioxygenases, dehydrogenases, metalloreductases, ferredoxins, thioredoxins);
2. Transferases (IUBMB EC 2: including, *e*.*g*., glycosyltransferases, alkyltransferases, acyltransferases, carboxyltransferases, fatty acyl synthases, kinases, RNA and DNA polymerases, reverse transcriptases, nucleic acid integrases);
3. Hydrolases (IUBMB EC 3: including, *e*.*g*., glycosylases, glycosidases, glucohydrolases, glucanases, amylases, cellulases, peptidases and proteases, nucleases, phosphatases, lipases, nucleic acid recombinases);
4. Lyases (IUBMB EC 4: including, *e*.*g*.., decarboxylases, RUBISCOs, adenylate cyclases);
5. Isomerases (IUBMB EC 5: including, *e*.*g*., racemases, epimerases, mutases, topoisomerases, gyrases, foldases); and
6. Ligases (IUBMB EC 6: including, *e*.*g*., carboxylases, acyl synthetases, peptide synthetases, nucleic acid ligases).

### Extremozymes

A wide range of extremozymes are known in the art. See, *e*.*g*., references 11-20. As used herein, the term "extremozyme" means an enzyme exhibiting an optimum of at least one catalytic property under at least one extremophilic condition as defined in Table 1, and encoded by either: 1) nucleic acid obtained from an extremophilic organism; or 2) nucleic acid obtained from an extremophilic organism and further altered by mutagenesis and/or recombination as described below. In a preferred embodiment, the extremophilic organism will be an extremophilic Archaeon, extremophilic bacterium, or extremophilic eukaryote. Particularly preferred extremophilic eukaryotes include extremophilic fungi and extremophilic yeasts. In a particularly preferred embodiment, the organism will be an extremophilic Archaeon or an extremophilic bacteria.

Whether the extremozyme-encoding nucleic acid is native or altered, the codons of the coding sequence(s) of the nucleic acid may be optimized according to the codon usage frequency of a host cell in which it is to be expressed. The catalytic property in which the optimum is exhibited may be, *e*.*g*.: catalytic activity per se or enzymatic throughput; a metric such as Kₘ, k_{cat}, kᵢ, kᵢᵢ, or Vₘₐₓ; or stability (catalytic half-life) under conditions of use or proposed use. In addition, the term "extremozyme," as used herein in reference to extremozyme expression systems of the present invention, is restricted to those extremozymes that are heterologous to a selected host cell chosen for expression thereof.

Nucleic acids encoding extremozymes may be obtained, *e*.*g*., directly from environmental samples using techniques commonly available in the art, *e*.*g*., the techniques described in: US Patent Nos. 5,958,672, 6,057,103, and 6,280,926 to Short; US Patent No. 6,261,842 to and WO 01/81567 of Handelsman et al.; US Patent No. 6,090,593 to Fleming & Sayler; or in L. Diels et al., Use of DNA probes and plasmid capture in a search for new interesting environmental genes, Sci. of the Total Environ. 139-140:471-8 (Nov 1, 1993). In addition, the techniques described in the following references may also be used: S. Jorgensen et al., in J. Biol. Chem. 272(26):16335-42 (Jun 27, 1997); EP Patent No. 577257B1 to Laderman & Anfinsen; EP Patent No. 579360B1 to Asada et al.; EP 648843A1 of Taguchi et al.; WO 98/45417 of Zeikus et al.; US Patent No. 6,100,073 to Deweer & Amory; and G. Dong et al., in Appl. Environ. Microbiol. 63(9):3577-84 (Sep 1997).

Once obtained, the extremozyme-encoding nucleic acids may be altered and expressed to obtain an extremozyme exhibiting improvement in or toward a desired catalytic property. Such alteration may be accomplished by use of one or more rounds of nucleic acid mutagenesis and/or recombination, resulting in formation of a library comprising altered nucleic acids, followed by (or, if desired when using multiple rounds, regularly or intermittently alternating with) expression of the library and screening of the enzymes. The nucleic acid mutagenesis and recombination technique(s) selected may be *in vitro* techniques or *in vivo* or *in cyto* techniques, and may be random techniques (random mutagenesis, random recombination) or directed techniques (*e*.*g*., oligonudeotide-directed mutagenesis, site-directed recombination). Many such mutagenesis and recombination techniques are commonly known in the art. For example, any of the techniques described in U.S. Patent Nos. 5,830,696, 5,965,408, or 6,171,820 to Short; in US Patent Nos. 5,605,793 and 5,811,238 to Stemmer et al.; and WO 98/42832 to Arnold et al. may be used; in addition, mutagenesis may be performed by use of the technique, Error-Prone PCR (also referred to as Low-Fidelity PCR).

In the present invention, the extremozyme is selected from among any of the enzymes within IUBMB EC 3, *i*.*e*. extremophilic hydrolases. In a preferred embodiment, the extremozyme will be selected from among any of the enzymes within IUBMB EC 3.1-3.8. In a preferred embodiment, the extremozyme will be selected from among any of the enzymes within IUBMB EC 3.1-3.2. In a preferred embodiment, the extremozyme will be selected from among any of the enzymes within IUBMB EC 3.2, *i*.*e*. extremophilic glycosylases. In a preferred embodiment, the extremozyme will be selected from among any of the enzymes within IUBMB EC 3.2.1, *i*.*e*. extremophilic glycosidases. In a preferred embodiment, the extremozyme will be selected from among any of the following enzymes within IUBMB EC 3.2.1: amylases, amyloglucosidases, and glucoamylases; cellulases, cellobiohydrolases, endoglucanases, and hemicellulases; and beta glucosidases. In a preferred embodiment, the extremozyme will be selected from among any of the following enzymes within IUBMB EC 3.2.1: amylases and cellulases. In a preferred embodiment, the extremozyme will be selected from among any of the amylases within IUBMB EC 3.2.1, *i*.*e*. extremophilic amylases. In a preferred embodiment, the extremozyme will be selected from among any of the alpha-amylases within IUBMB EC 3.2.1 (*i*.*e*., the enzymes of IUBMB EC 3.2.1.1), thus, the extremophilic alpha-amylases.

In a preferred embodiment, the extremozyme will be selected from among any of the enzymes within IUBMB EC 3.4. In a preferred embodiment, the extremozyme will be selected from among any of the enzymes within IUBMB EC 3.4.21 or 3.4.23, *i*.*e*. extremophilic serine peptidases and extremophilic aspartic endopeptidases. In a preferred embodiment, the extremozyme will be selected from among any of the following enzymes within IUBMB EC 3.4.21 and 3.4.23: pyrolysins and thermopsins.

In a preferred embodiment, the extremozyme is at least one of: hyperthermophilic, psychrophilic, acidophilic, alkalophilic, and halophilic. In a preferred embodiment, the extremozyme is at least one of: hyperthermophilic, psychrophilic, acidophilic, and alkalophilic. In a preferred embodiment, the extremozyme is at least one of: hyperthermophilic, acidophilic, and alkalophilic. In a preferred embodiment, the extremozyme is at least hyperthermophilic. Particularly preferred are at least hyperthermophilic extremozymes.

In the extremozyme expression systems of the present invention, the extremozyme-encoding nucleic acid will be operably linked to a control sequence, and optionally other element(s), to form an expression construct (also called an "expression cassette"), and the resulting expression construct will be inserted into an expression vector; alternatively, the expression cassette can be constructed within the vector by inserting the elements of the expression cassette into the vector in any other series of steps. The expression vector will be then be transformed into a bacterial host cell according to the present invention, followed by expression of the extremozyme.

### Vectors

A great many bacterial vectors are known in the art as useful for expressing proteins in the Gram(-) Proteobacteria, and these may be used for expressing the extremozymes according to the present invention. Such vectors include, *e*.*g*., plasmids, cosmids, and phage expression vectors. Examples of useful plasmid vectors include the expression plasmids pMB9, pBR312, pBR322, pML122, RK2, RK6, and RSF1010. Other examples of such useful vectors include those described by, *e*.*g*.: N Hayase, in Appl. Envir. Microbiol. 60(9):3336-42 (Sep 1994); AA Lushnikov et al., in Basic Life Sci. 30:657-62 (1985); S Graupner & W Wackernagel, in Biomolec. Eng. 17(1):11-16. (Oct 2000); HP Schweizer, in Curr. Opin. Biotech. 12(5):439-45 (Oct 2001); M Bagdasarian & KN Timmis, in Curr. Topics Microbiol. Immunol. 96:47-67 (1982); T Ishii et al., in FEMS Microbiol. Lett. 116(3):307-13 (Mar 1, 1994); IN Olekhnovich & YK Fomichev, in Gene 140(1):63-65 (Mar 11, 1994); M Tsuda & T Nakazawa, in Gene 136(1-2):257-62 (Dec 22, 1993); C Nieto et al., in Gene 87(1):145-49 (Mar 1, 1990); JD Jones & N Gutterson, in Gene 61(3):299-306 (1987); M Bagdasarian et al., in Gene 16(1-3):237-47 (Dec 1981); HP Schweizer et al., in Genet. Eng. (NY) 23:69-81 (2001); P Mukhopadhyay et al., in J. Bact. 172(1):477-80 (Jan 1990); DO Wood et al., in J. Bact. 145(3):1448-51 (Mar 1981); and R Holtwick et al., in Microbiology 147(Pt 2):337-44 (Feb 2001).

Further examples of useful *Pseudomonas* expression vectors include those listed in Table 2.

| Table 2. Some Examples of Useful Expression Vectors, Promoters, and Inducers | | | | |
|---|---|---|---|---|
| Replicon | Vector(s) | Promoter | Inducer | Reference |
| pPS10 | PCN39, pCN51 | None | | 1 |
| RSF1010 | PKT261-3 | | | 2 |
| | PMMB66EH | P_{tac} | IPTG | 3 |
| | PEB8 | P_{T7} | IPTG | 4 |
| | PPLGNI | λ_{PR} | Temperature | 5 |
| | PERD20/21 | Pₘ | Benzoate | 6 |
| RK2/RP1 | PRK415 | | | 7 |
| | PJB653 | | | 8 |
| pRO1600 | PUCP | | | 9 |
| | PBSP | | | 10 |

The expression plasmid, RSF1010, is described, *e*.*g.*, by F Heffron et al., in Proc. Nat'l Acad. Sci. USA 72(9):3623-27 (Sep 1975), and by K Nagahari & K Sakaguchi, in J. Bact. 133(3):1527-29 (Mar 1978). Plasmid RSF1010 and derivatives thereof are particularly useful vectors in the present invention. Exemplary, useful derivatives of RSF1010, which are known in the art, include, *e*.*g*., pKT212, pKT214, pKT231 and related plasmids, and pMYC1050 and related plasmids (see; *e*.*g*., US Patent Nos. 5,527,883 and 5,840,554 to Thompson et al.), such a, *e*.*g*., pMYC1803. Other particularly useful vectors include those described in US Patent No. 4,680,264 to Puhler et al.

In a preferred embodiment, an expression plasmid is used as the expression vector. In a preferred embodiment, RSF1010 or a derivative thereof is used as the expression vector. In a preferred embodiment, pMYC1050 or a derivative thereof, or pMYC 1803 or a derivative thereof, is used as the expression vector.

### Control Sequences

The term "control sequence" is defined herein as the set of all elements which are necessary, and optionally other elements that are advantageous, for the expression of an extremozyme in the host cells according to the present invention. Each control sequence element may be native or foreign to the nucleic acid encoding the extremozyme and may be native or foreign to the host cell. Such control sequence elements include, but are not limited to: promoters; transcriptional enhancers; ribosome binding sites (also called "Shine Delgarno sequences"); translational enhancers (see, *e*.*g*., US Patent No. 5,232,840 to Olins); leader peptide-encoding sequences, *e*.*g*., for targeting peptides or secretion signal peptides, propeptide-coding sequences; transcriptional and translational start and stop signals, polyadenylation signals; and transcription terminators.

At a minimum, the control sequence(s) will include a promoter, a ribosome binding site, and transcriptional and translational start and stop signals and a transcription terminator. The control sequence elements, vector, and extremozyme coding sequence may be attached to, or extended to add, linkers or tails for the purpose of introducing specific sequences (*e*.*g*., restriction sites) facilitating assembly (*e*.*g*., *via* ligation, recombination, or PCR overlap extension) of the control sequence elements with the coding sequence(s) of the nucleic acid encoding an extremozyme, and with the vector. The term "operably linked," as used herein, refers to any configuration in which the elements of the control sequence are covalently attached to the coding sequence in such disposition(s), relative to the coding sequence, that in and by action of the host cell, the control sequence can direct the expression of the coding sequence.

### Promoters

The promoter may be any nucleic acid sequence that exhibits transcriptional activity in the host cell of choice, and may be a native, mutant, truncated, or hybrid promoter; native promoters may be obtained from polypeptide-encoding genes that are either native or heterologous to the host cell. If desired, the nucleic acid containing the promoter may remain linked to a ribosome binding site found attached thereto, and optionally to at least part of the coding sequence controlled thereby, as found in its native configuration. (This native coding sequence or portion thereof, if retained, will be attached to the extremozyme coding sequence, ultimately resulting in expression of an extremozyme-fusion protein.)

Any of the many promoters known in the art as capable of directing transcription in the host cells of the present invention may be selected for use therein. See, *e*.*g*., Sambrook *et al*. *(1989), supra.* The promoter selected may be either a constitutive promoter or a regulated promoter, provided that where the extremozyme is expressed intracellularly (*i*.*e*., where it is not secreted or otherwise delivered to a point beyond the host cell's cytoplasm) a constitutive promoter is preferably not used.

Where a regulated promoter is selected, it may be either a positively or negatively regulated promoter. A positively regulated promoter is one that is regulated, via transcriptional activation by an activator protein, to begin transcribing mRNA upon induction. A negatively regulated promoter is one that is repressed by a repressor protein and which permits transcription of mRNA only upon de-repression upon induction. Either a reversibly-inducible or irreversibly-inducible regulated promoter may be selected.

Where a positively regulated promoter is used, the expression system will also contain, or will be genetically engineered to contain, a gene encoding an activator protein therefor, which gene is expressed, preferably constitutively expressed, in the host cell. The activator protein-encoding gene is preferably contained within the host cell chromosome, or it may be contained on the same vector as, or a different vector from, the vector containing the extremozyme-encoding nucleic acid). Many such positively regulated promoters and positively regulated promoter-activator protein combinations are know in the art. For example, see: US Patent Nos. 5,670,350, 5,686,283, and 5,710,031 to Gaffney et al.; US Patent No. 5,686,282 to Lam et al.; Albright et al., in Annual Rev. Genet. 23:311-336 (1989); Bourret et al., in Annual Rev. Biochem. 60:401-441 (1991); and Mekalanos, J. Bact. 174:1-7 (1992).

Examples of positively regulated promoters include, *e*.*g*.: the "meta promoter" (Pₘ) from the meta operon of the toluene-catabolic-pathway-encoding plasmid pWW0 of *Pseudomonas putida* (see N Hugouvieux-Cotte-Pattat et al., in J. Bact. 172(12):6651-60 (Dec 1990)); and the araB promoter, which is inducible by addition of L-arabinose which interacts with the activator (the product of the araC gene), as described in US Patent No. 5,028,530.

Where a negatively regulated promoter is used, the expression system will also contain, or will be genetically engineered to contain, a gene encoding a repressor protein therefor, which gene is expressed, preferably constitutively expressed, in the host cell. The repressor-protein-encoding gene may be contained on the same vector as, or a different vector from, the vector containing the extremozyme-encoding nucleic acid (or it may be contained within the host cell chromosome). Examples of useful repressors, and genes encoding them, include those described in US Patent Nos. 5,210,025 and 5,356,796 to Keller.

Many negatively regulated promoters and negatively regulated promoter-repressor combinations are well known in the art. Examples of preferred negatively regulated promoters include the *E. coli* tryptophan promoter (Pₜᵣₚ), the *E. coli* lactose promoter (P_{lac}) and derivatives thereof (*e*.*g*., the tac, tacII, and trc promoters, P_{tac}, P_{tacII}, and P_{trc}, described in US Patent No. 4,551,433 to DeBoer), the phage T7 promoter (P_{T7}), lambda phage promoters (*e*.*g*., λ_{PL}, λ_{PR}), and the recA promoter from *Rhodobacter capsulatus*. All of the P_{lac}, P_{lac}, P_{lacII}, P_{trc}, and P_{T7} promoters are repressed by the lac repressor (lacI).

Where a regulated promoter is used, at an appropriate time during the host cell growth cycle, an inducer will be added to activate or de-repress the regulated promoter. Many positively regulated promoter-activator protein-inducer combinations and many negatively regulated promoter-repressor protein-inducer combinations, effective in the host cells of the present invention are well known in the art. For example, in the case of Pₘ, benzoate will serve as an inducer; and in the case of P_{lac}, P_{tac}, P_{tacII}, P_{trc}, and P_{T7}, one preferred inducer is IPTG. Also see Table 2. Where an extremozyme is expressed intracellularly within the host cell, preferably the inducer for the regulated promoter will be added upon, or shortly prior to, achievement of maximum host cell proliferation, *i*.*e*. maximum "cell density." Especially preferred is to add the inducer at about the mid-log phase of cell proliferation.

In a preferred embodiment of the present invention, a regulated promoter is selected. In a preferred embodiment, a positively regulated promoter is selected, preferably Pₘ. In a preferred embodiment, a negatively regulated promoter is selected, preferably P_{tac}. In a preferred embodiment, a negatively regulated promoter is selected for use in an intracellular extremozyme expression system according to the present invention. In a preferred embodiment, the negatively regulated, promoter is P_{tac} and the promoter-repressor-inducer combination in which the regulated promoter is utilized will be P_{tac}-lacI-IPTG.

A secreted protein expression system can use either constitutive or regulated promoters. In a secreted protein expression system, either an extremozyme or an extremozyme-fusion protein is secreted from the host cell. A regulated promoter for a secreted protein expression system can be selected from, *e*.*g*., any of those regulated promoters described above. A constitutive promoter for a secreted protein expression system can be selected from among any of the large number of constitutive promoters known in the art as effective for protein expression in the host cells of the present invention. A particularly useful constitutive promoter is the neomycin phosphotransferase II promoter (P_{nptII}) obtained from transposon Tn5. See, *e*.*g*., DW Bauer & A Collmer, Mol. Plant Microbe Interact. 10(3):369-79 (Apr 1997); and C Casavant et al., A novel genetic system to direct programmed, high-level gene expression in natural environments, Abstracts of the 99th American Society for Microbiology General Meeting (held May 30 - June 3,1999 in Chicago, IL, USA). In a preferred embodiment of a secreted protein expression system, a constitutive promoter is used; in a preferred embodiment of a secreted protein expression system, P_{nptII} is used as the promoter for the extremozyme-encoding nucleic acid.

### Other Elements and Methods

Other elements may also be included within the expression system according to the present invention. For example, a tag sequence that facilitates identification, separation, purification, or isolation of an extremozyme expressed as a fusion protein therewith can be encoded by a coding sequence attached to the coding sequence of the extremozyme. In a preferred embodiment of the present invention, where use of a tag sequence is desired, the tag sequence is a hexa-histidine peptide and the extremozyme coding sequence is fused to a hexa-histidine-encoding sequence. Similarly, the extremozyme may be expressed as a fusion protein with a whole or partial viral structural protein, *e*.*g*., a viral (or phage) coat protein, by attaching all or part of the viral coat protein coding sequence to the coding sequence of the extremozyme.

Furthermore, one or more marker genes or reporter genes may be used in the expression system to verify expression of the extremozyme. Many such useful marker or reporter genes are known in the art. See, *e.g.,* US Patent No. 4,753,876 to Hemming et al.*,* and DL Day et al., in J. Bact. 157(3):937-39 (Mar 1984). In a preferred embodiment, the marker gene is selected from among the antibiotic resistance-conferring marker genes. In a preferred embodiment, the marker gene is selected from among the tetracycline and kanamycin resistance genes. In a preferred embodiment, a reporter gene is selected from among those encoding: (1) fluorescent proteins (*e*.*g*., GFP); (2) colored proteins; and (3) fluorescence- or color-facilitating or -inducing proteins, the latter class (3) including, *e*.*g*., luminases and beta-galactosidese genes. Beta-galactosidases hydrolze X-gal to create a blue-colored derivative.

Further examples of methods, vectors, and translation and transcription elements, and other elements useful in the present invention are described in, *e*.*g*.: US Patent No. 5,055,294 to Gilroy and US Patent No. 5,128,130 to Gilroy et al.; US Patent No. 5,281,532 to Rammier *et al*.; US Patent Nos. 4,695,455 and 4,861,595 to Barneys et al.; US Patent No. 4,755,465 to Gray et al.; and US Patent No. 5,169,760 to Wilcox.

### Host Cells

Whether in native or altered form, the extremozyme-encoding nucleic acids will be over-expressed, according to the present invention, in bacterial host cells selected from Pseudomonads.

In a preferred embodiment, the host cell is selected from Proteobacteria of the following *Pseudomonas* species (with the ATCC or other deposit numbers of exemplary strain(s) shown in parenthesis): *Pseudomonas abietaniphila* (ATCC 700689); *Pseudomonas aeruginosa* (ATCC 10145); *Pseudomonas alcaligenes* (ATCC 14909); *Pseudomonas anguilliseptica* (ATCC 33660); *Pseudomonas citronellolis* (ATCC 13674); *Pseudomonas flavescens* (ATCC 51555); *Pseudomonas mendocina* (ATCC 25411); *Pseudomonas nitroreducens* (ATCC 33634); *Pseudomonas oleovorans* (ATCC 8062); *Pseudomonas pseudoalcaligenes* (ATCC 17440); *Pseudomonas resinovorans* (ATCC 14235); *Pseudomonas straminea* (ATCC 33636); *Pseudomonas agarici* (ATCC 25941); *Pseudomonas alcaliphila; Pseudomonas alginovora; Pseudomonas andersonii*; *Pseudomonas asplenii* (ATCC 23835); *Pseudomonas azelaica* (ATCC 27162); *Pseudomonas beijerinckii* (ATCC 19372); *Pseudomonas borealis; Pseudomonas boreopolis* (ATCC 33662); *Pseudomonas brassicacearum; Pseudomonas butanovora* (ATCC 43655); *Pseudomonas cellulosa* (ATCC 55703); *Pseudomonas aurantiaca* (ATCC 33663); *Pseudomonas chlororaphis* (ATCC 9446, ATCC 13985, ATCC 17418, ATCC 17461); *Pseudomonas fragi* (ATCC 4973); *Pseudomonas lundensis* (ATCC 49968); *Pseudomonas taetrolehs* (ATCC 4683); *Pseudomonas cissicola* (ATCC 33616); *Pseudomonas coronafaciens; Pseudomonas diterpeniphila; Pseudomonas elongata* (ATCC 10144); *Pseudomonas flectens* (ATCC 12775); *Pseudomonas azotoformans; Pseudomonas brenneri*; *Pseudomonas cedrella; Pseudomonas corrugata* (ATCC 29736); *Pseudomonas extremorientalis; Pseudomonas fluorescens* (ATCC 35858); *Pseudomonas gessardii; Pseudomonas libanerisis; Pseudomonas mandelii* (ATCC 700871); *Pseudomonas marginalis* (ATCC 10844*); Pseudomonas migulae; Pseudomonas mucidolens* (ATCC 4685); *Pseudomonas orientalis; Pseudomonas rhodesiae*; *Pseudomonas synxantha* (ATCC *9890); Pseudomorias tolaasii* (ATCC 33618); *Pseudomonas veronii* (ATCC 700474); *Pseudomonas frederiksbergensis*; *Pseudomonas geniculata* (ATCC 19374); *Pseudomonas gingeri; Pseudomonas graminis; Pseudomonas grimontii; Pseudomonas halodenitrificans; Pseudomonas halophila*; *Pseudomonas hibiscicola* (ATCC 19867); *Pseudomonas huttiensis* (ATCC 14670); *Pseudomonas hydrogenovora; Pseudomonas jessenii* (ATCC 700870); *Pseudomonas kilonensis; Pseudomonas lanceolata* (ATCC 14669); *Pseudomonas lini; Pseudomonas marginata* (ATCC 25417); *Pseudomonas mephitica* (ATCC 33665); *Pseudomonas denitrificans* (ATCC 19244); *Pseudomonas pertucinogena* (ATCC 190); *Pseudomonas pictorum* (ATCC 23328); *Pseudomonas psychrophila; Pseudomonas fulva* (ATCC 31418); *Pseudomonas monteilii* (ATCC 700476); *Pseudomonas mosselii; Pseudomonas oryzihabitans* (ATCC 43212); *Pseudomonas plecoglossicida* (ATCC 700383); *Pseudomonas putida* (ATCC 12633); *Pseudomonas reactans; Pseudomonas spinosa* (ATCC 14606); *Pseudomonas balearica; Pseudomonas luteola* (ATCC 43273); *Pseudomonas stutzeri* (ATCC 17588); *Pseudomonas amygdali* (ATCC 33614); *Pseudomonas avellanae* (ATCC 700331); *Pseudomonas caricapapayae* (ATCC 33615); *Pseudomonas cichorii* (ATCC 10857); *Pseudomonas ficuserectae* (ATCC 35104); *Pseudomonas fuscovaginae; Pseudomonas meliae* (ATCC 33050); *Pseudomonas syringae* (ATCC 19310); *Pseudomonas viridiflava* (ATCC 13223); *Pseudomonas thermocarboxydovorans* (ATCC 35961); *Pseudomonas thermotolerans; Pseudomonas thivervalensis; Pseudomonas vancouverensis* (ATCC 700688); *Pseudomonas wisconsinensis;* and *Pseudomonas xiamenensis*.

In a preferred embodiment, the host cell is selected from Proteobacteria known in the art as the "fluorescent Pseudomonads" including those belonging, *e*.*g*., to the following *Pseudomonas* species: *Pseudomonas azotoformans; Pseudomonas brenneri; Pseudomonas cedrella; Pseudomonas corrugata; Pseudomonas extremorientalis; Pseudomonas Jluorescens; Pseudomonas gessardii; Pseudomonas libanensis; Pseudomonas mandelii; Pseudomonas marginalis*; *Pseudomonas migulae; Pseudomonas mucidolens; Pseudomonas orientalis; Pseudomonas rhodesiae*; *Pseudomonas synxantha*; *Pseudomonas tolaasii*; and *Pseudomonas veronii.*

In a preferred embodiment, the host cell is selected from all subspecies, varieties, strains, and other sub-special units of the species *Pseudomonas fluorescens*, including those belonging, *e*.*g*., to the following (with the ATCC or other deposit numbers of exemplary strain(s) shown in parenthesis): *Pseudomonas fluorescens* biotype A, also called biovar 1 or biovar I (ATCC 13525); *Pseudomonas fluorescens* biotype B, also called biovar 2 or biovar II (ATCC 17816); *Pseudomonas fluorescens* biotype C, also called biovar 3 or biovar III (ATCC 17400); *Pseudomonas fluorescens* biotype F, also called biovar 4 or biovar IV (ATCC 12983); *Pseudomonas fluorescens* biotype G, also called biovar 5 or biovar V (ATCC 17518); and *Pseudomonas fluorescens* subsp. *cellulosa* (NCIMB 10462).

In a preferred embodiment, the host cell is selected from all strains of *Pseudomonas fluorescens* biotype A. A particularly preferred strain of this biotype is *P. fluorescens* strain MB101 (see US Patent No. 5,169,760 to Wilcox), and derivatives thereof.

### Transformation

Transformation of the host cells with the vector(s) may be performed using any transformation methodology known in the art, and the bacterial host cells may be transformed as intact cells or as protoplasts (*i*.*e*. including cytoplasts). Exemplary transformation methodologies include poration methodologies, *e*.*g*., electroporation, protoplast fusion, bacterial conjugation, and divalent cation treatment, *e*.*g*., calcium chloride treatment or CaCl/Mg²⁺ treatment.

### Fermentation

As used herein, the term "fermentation" includes both embodiments in which literal fermentation is employed and embodiments in which other, non-fermentative culture modes are employed. Fermentation may be performed at any scale. In a preferred embodiment, The fermentation medium may be selected from among rich media, minimal media, and mineral salts media; a rich medium may be used, but is preferably avoided. In a preferred embodiment either a minimal medium or a mineral salts medium is selected.

In a preferred embodiment, a minimal medium is selected. In a preferred embodiment, a mineral salts medium is selected. Mineral salts media are particularly preferred.

Mineral salts media consist of mineral salts and a carbon source such as, *e*.*g*., glucose, sucrose, or glycerol. Examples of mineral salts media include, *e*.*g.*, M9 medium, *Pseudomonas* medium (ATCC 179), Davis and Mingioli medium (see, BD Davis & ES Mingioli, in J. Bact. 60:17-28 (1.950)). The mineral salts used to make mineral salts media include those selected from among, *e*.*g*., potassium phosphates, ammonium sulfate or chloride, magnesium sulfate or chloride, and trace minerals such as calcium chloride, borate, and sulfates of iron, copper, manganese, and zinc. No organic nitrogen source, such as peptone, tryptone, amino acids, or a yeast extract, is included in a mineral salts medium. Instead, an inorganic nitrogen source is used and this may be selected from among, *e*.*g*., ammonium salts, aqueous ammonia, and gaseous ammonia. A preferred mineral salts medium will contain glucose as the carbon source. In comparison to mineral salts media, minimal media also contain mineral salts and a carbon source, but are further supplemented with, *e*.*g*., low levels of amino acids, vitamins, peptones, or other ingredients, though these are added at very minimal levels.

The extremozyme expression system according to the present invention can be cultured in any fermentation format. For example, batch, fed-batch, semi-continuous, and continuous fermentation modes may be employed herein.

The expression systems according to the present invention are useful for extremozyme expression at any scale (*i*.*e*. volume) of fermentation Thus, *e*.*g*., microliter-scale, centiliter scale, and deciliter scale fermentation volumes may be used; and 1 Liter scale and larger fermentation volumes can be used. In a preferred embodiment, the fermentation volume will be at or above 1 Liter. In a preferred embodiment, the fermentation volume will be at or above 5 Liters. In a preferred embodiment, the fermentation volume will be at or above 10 Liters. In a preferred embodiment, the fermentation volume will be at or above 15 Liters. In a preferred embodiment, the fermentation volume will be at or above 20 Liters. In a preferred embodiment, the fermentation volume will be at or above 25 Liters. In a preferred embodiment, the fermentation volume will be at or above 50 Liters. In a preferred embodiment, the fermentation volume will be at or above 75 Liters. In a preferred embodiment, the fermentation volume will be at or above 100 Liters. In a preferred embodiment, the fermentation volume will be at or above 150 Liters. In a preferred embodiment, the fermentation volume will be at or above 200 Liters. In a preferred embodiment, the fermentation volume will be at or above 250 Liters. In a preferred embodiment, the fermentation volume will be at or above 500 Liters. In a preferred embodiment, the fermentation volume will be at or above 750 Liters. In a preferred embodiment, the fermentation volume will be at or above 1,000 Liters. In a preferred embodiment, the fermentation volume will be at or above 2,000 Liters. In a preferred embodiment, the fermentation volume will be at or above 2,500 Liters. In a preferred embodiment, the fermentation volume will be at or above 5,000 Liters. In a preferred embodiment, the fermentation volume will be at or above 10,000 Liters. In a preferred embodiment, the fermentation volume will be at or above 50,000 Liters. In a particularly preferred embodiment, the fermentation volume will be at or above 10 Liters.

In the present invention, growth, culturing, and/or fermentation of the host cells is performed within a temperature range of about 4°C to about 55°C, inclusive. Thus, *e.g*., the terms "growth" (and"grow," "growling"), "culturing" (and "culture"), and "fermentation" (and "ferment," "fermenting"), as used herein in regard to the host cells of the present invention, inherently and necessarily means "growth," "culturing," and "fermentation," within a temperature range of about 4°C to about 55°C, inclusive. In addition, "growth" is used to indicate both biological states of active cell division and/or enlargement, as well as biological states in which a non-dividing and/or non-enlarging cell is being metabolically sustained, the latter use of the term "growth" being synonymous with the term "maintenance."

In addition, growth "under conditions permitting expression" when used in regard to the recombinant bacterial host cells and expression systems of the present invention, is defined herein to mean: (1) growth of the recombinant bacterial host cells per se, where the promoter used in the control sequence operably linked to the extremozyme coding sequence is a constitutive promoter; and (2) where the promoter used in the control sequence operably linked to the extremozyme coding sequence is a regulated promoter, (a) growth of the recombinant bacterial host cells in the presence of (*i.e*. in contact with) an inducer therefor, and (b) growth of the recombinant bacterial host cells in the absence of an inducer therfor, followed by addition of such an inducer to the system, thereby causing contact between the cell and the inducer.

### Biocatalyst Preparation

Once expressed, the extremozymes can then be separated, isolated, and/or purified using any protein recovery and/or protein purification methods known in the art. For example, where the extremozyme is expressed intracellularly, the host cell can be lysed by standard physical, chemical, or enzymatic means, see, *e.g.,* P. Prave et al. (eds.), Fundamentals of Biotechnology (1987) (VCH Publishers, New York) (especially Section 8.3), following by separation of the proteins, *e.g*., by any one or more of microfiltration, ultrafiltration, gel filtration, gel purification (e.g., by PAGE), affinity purification, chromatography (e.g., LC, HPLC, FPLC), and the like. Alternatively, variations of these commonly known protein recovery and protein purification methods can be used which capitalize on the specific properties of these enzymes. For example, it has been reported that hyperthermophilic enzymes can be easily separated from cellular materials by heating which resuspends the extremozymes while causing precipitation of the other cellular proteins and materials; this method is particularly preferred for use with hyperthermophilic enzymes herein.

Where the extremozyme is secreted from the host cell, it can be directly separated, isolated, and/or purified from the medium. Where the extremozyme is expressed in the host cell as, or as part of, an insoluble inclusion body, the inclusion body will be solubilized to permit recovery of functional enzymes. For example, the host cells can be lysed to obtain such inclusion bodies therefrom, and then solubilized; alternatively, some extremozyme inclusion bodies can be directly extracted from the host cell by solubilization *in cyto* without use of a cell lysis step. In either embodiment, such solubilization may result in some degree of unfolding of the expressed extremozyme. Where solubilization results in unfolding of the expressed extremozyme, a refolding step will preferably follow the solubilization step.

Various techniques for solubilizing and refolding the enzymes and other proteins expressed in inclusion bodies are known in the art. See, for example: E De Bernardez Clark, Protein refolding for industrial processes, Curr. Opin. in Biotechnol. 12(2):202-07 (Apr 1, 2001); MM Carrió & A Villaverde, Protein aggregation as bacterial inclusion bodies is reversible, FEBS Lett. 489(1):29-33 (Jan 26, 2001); R Rudolph & H Lilie, In vitro folding of inclusion body proteins, FASEB J. 10:49-56 (1996); B Fischer et al., in Biotechnol. Bioeng. 41:3-13 (1993) (refolding of eukaryotic proteins expressed in *E. coli*); G. Dong et al., in Appl. Envir. Microbiol. 63(9):3569-3576 (Sep 1997) (refolding of an extremophilic amylase enzyme); A Yamagata et al., in Nucl. Acids Res., 29(22):4617-24 (Nov 15, 2001) (urea denaturation to solubilize a heterologous, thermophilic RecJ exonuclease enzyme, followed by refolding to obtain an active enzyme); and C Pire et al., in FEMS Microbiol. Lett. 200(2):221-27 (Jun 25, 2001) (refolding of an archaeal halophilic glucose dehydrogenase expressed in *E. coli*).

The extremozyme expressed according to the present invention can be used in a biocatalytic process, such as described above. Preferred biocatalytic processes are industrial biocatalytic processes. Once separated, isolated, or purified, the extremozymes can then be used to perform biocatalysis, *e*.*g*., in free-enzyme or immobilized-enzyme bioreactors, *e*.*g*., in place of current industrial enzymes. Alternatively, once the extremozyme has been expressed (or while it is being expressed) by the host cell, it can be used *in cyto* for biocatalysis. For example, the cell can be used as a biocatalytic unit, *e*.*g*., in a whole-cell bioreactor, whether a free-cell or immobilized-cell bioreactor; in this format, the extremozyme can be expressed intracellularly or on the cell surface or can be secreted or otherwise exported from the cell. In a preferred embodiment using this format, the extremozyme is expressed either intracellularly or on the cell surface. The resulting enzyme or whole-cell bioreactor can itself be a batch, fed-batch, semi-continuous, or continuous bioreactor.

### Expression Levels

The expression systems according to the present invention express extremozymes at a level at or above 5% tcp. In a preferred embodiment, the expression level will be at or above 8% tcp. In a preferred embodiment, the expression level will be at or above 10% tcp. In a preferred embodiment, the expression level will be at or above 15% tcp. In a preferred embodiment, the expression level will be at or above 20% tcp. In a preferred embodiment, the expression level will be at or above 25% tcp. In a preferred embodiment, the expression level will be at or above 30% tcp. In a preferred embodiment, the expression level will be at or above 40% tcp. In a preferred embodiment, the expression level will be at or above 50% tcp.

In a preferred embodiment, the expression level will be at or below 35% tcp. In a preferred embodiment, the expression level will be at or below 40% tcp. In a preferred embodiment, the expression level will be at or below 45% tcp. In a preferred embodiment, the expression level will be at or below 50% tcp. In a preferred embodiment, the expression level will be at or below 60% tcp. In a preferred embodiment, the expression level will be at or below 70% tcp. In a preferred embodiment, the expression level will be at or below 80% tcp.

In a preferred embodiment, the expression level will be between 5% tcp and 80% tcp. In a preferred embodiment, the expression level will be between 8% tcp and 70% tcp, inclusive. In a preferred embodiment, the expression level will be between 10% tcp and 70% tcp, inclusive. In a preferred embodiment, the expression level will be between 15% tcp and 70% tcp, inclusive. In a particularly preferred embodiment, the expression level will be between 20% tcp and 70% tcp, inclusive.

### Cell Density

The expressions systems according to the present invention provide a cell density, *i*.*e*. a maximum cell density, of at least about 20 g/L (even when grown in mineral salts media); the expressions systems according to the present invention likewise provide a cell density of at least about 70 g/L, as stated in terms of biomass per volume, the biomass being measured as dry cell weight.

In a preferred embodiment, the cell density will be at least 20 g/L. In a preferred embodiment, the cell density will be at least 25 g/L. In a preferred embodiment, the cell density will be at least 30 g/L. In a preferred embodiment, the cell density will be at least 35 g/L. In a preferred embodiment, the cell density will be at least 40 g/L. In a preferred embodiment, the cell density will be at least 45 g/L. In a preferred embodiment, the cell density will be at least 50 g/L. In a preferred embodiment, the cell density will be at least 60 g/L. In a preferred embodiment, the cell density will be at least 70 g/L. In a preferred embodiment, the cell density will be at least 80 g/L. In a preferred embodiment, the cell density will be at least 90 g/L. In a preferred embodiment, the cell density will be at least 100 g/L. In a preferred embodiment, the cell density will be at least 110 g/L. In a preferred embodiment, the cell density will be at least 120 g/L. In a preferred embodiment, the cell density will be at least 130 g/L. In a preferred embodiment, the cell density will be at least 140 g/L. In a preferred embodiment, the cell density will be at least 150 g/L.

In a preferred embodiment, the cell density will be at or below 150 g/L. In a preferred embodiment, the cell density will be at or below 140 g/L. In a preferred embodiment, the cell density will be at or below 130 g/L. In a preferred embodiment, the cell density will be at or below 120 g/L. In a preferred embodiment, the cell density will be at or below 110 g/L. In a preferred embodiment, the cell density will be at or below 100 g/L. In a preferred embodiment, the cell density will be at or below 90 g/L. In a preferred embodiment, the cell density will be at or below 80 g/L. In a preferred embodiment, the cell density will be at or below 75 g/L. In a preferred embodiment, the cell density will be at or below 70 g/L.

In a preferred embodiment, the cell density will be between 20 g/L and 150 g/L, inclusive. In a preferred embodiment, the cell density will be between 20 g/L and 120 g/L, inclusive. In a preferred embodiment, the cell density will be between 20 g/L and 80 g/L, inclusive. In a preferred embodiment, the cell density will be between 25 g/L and 80 g/L, inclusive. In a preferred embodiment, the cell density will be between 30 g/L and 80 g/L, inclusive. In a preferred embodiment, the cell density will be between 35 g/L and 80 g/L, inclusive. In a preferred embodiment, the cell density will be between 40 g/L and 80 g/L, inclusive. In a preferred embodiment, the cell density will be between 45 g/L and 80 g/L, inclusive. In a preferred embodiment, the cell density will be between 50 g/L and 80 g/L, inclusive. In a preferred embodiment, the cell density will be between 50 g/L and 75 g/L, inclusive. In a preferred embodiment, the cell density will be between 50 g/L and 70 g/L, inclusive. In a particularly preferred embodiment, the cell density will be at least 40 g/L. In a particularly preferred embodiment, the cell density will be between 40 g/L and 80 g/L.

### Total Productivity

In the expression systems according to the present invention, the total productivity, i.e. the total extremozyme productivity, is at least 1 g/L. The factors of cell density and expression level are selected accordingly. In a preferred embodiment, the total productivity will be at least 2 g/L. In a preferred embodiment, the total productivity will be at least 3 g/L. In a preferred embodiment, the total productivity will be at least 4 g/L. In a preferred embodiment, the total productivity will be at least 5 g/L. In a preferred embodiment, the total productivity will be at least 6 g/L. In a preferred embodiment, the total productivity will be at least 7 g/L. In a preferred embodiment, the total productivity will be at least 8 g/L. In a preferred embodiment, the total productivity will be at least 9 g/L. In a preferred embodiment, the total productivity will be at least 10 g/L.

In a particularly preferred embodiment, the expression system will have an extremozyme expression level of at least 5% tcp and a cell density of at least 40 g/L, when grown (*i*.*e*. within a temperature range of about 4°C to about 55°C, inclusive) in a mineral salts medium. In a particularly preferred embodiment, the expression system will have an extremozyme expression level of at least 5% tcp and a cell density of at least 40 g/L, when grown (*i*.*e*. within a temperature range of about 4°C to about 55°C, inclusive) in a mineral salts medium at a fermentation scale of at least 10 Liters.

### EXAMPLES

### Example 1. Extremophilic Cellulase

Example 1A. Construction of *Pseudomonas fluorescens* strains expressing *Thermotoga maritima* and *Pyrococcus furiosus* cellulases.

### Methods

Molecular Biology techniques were as described in Ausubel et al. (eds.), Current Protocols in Molecular Biology (1995) (John Wiley & Sons); Sambrook, Fritsch, & Maniatis (eds.), Molecular Cloning (1989) (Cold Spring Harbor Laboratory Press, NY).

### Expression Cassettes

The parent plasmid pMYC1803 is a derivative of pTJS260 (see US Patent No. 5,169,760 to Wilcox), carrying a regulated tetracycline resistance marker and, the replication and mobilization loci from RSF1010 plasmid. (pMYC1803 is a source for many derivative plasmids useful in expression extremozymes according to the present invention. Most such derivatives differ from pMYC1803 primarily around the ORF in order to introduce convenient restriction sites for cloning different exogenous genes.)

The *Thermotoga maritima* cellulase gene (0.94 kb encoding the 314 aa, 38kD cellulase) and the *Pyrococcus furiosus* endoglucanase gene (0.90 kb encoding the 300 aa, 34kD endoglucanase) were PCR-amplified using primers designed to introduce a *Spe*I site at the N-terminal end, along with the translational start site of the ORF in pMYC1803, and a *Xho*I site at the C-terminus of the coding sequences of the genes. The *Spe*I - *Xho*I fragment of the respective PCR products were independently inserted into pMYC1803 at the corresponding sites such that the enzyme genes replaced an exogenous gene already present therein hence, their expression was initiated from the *tac* promoter. The resulting constructs, pMYC1954 and pDOW2408, in *E. coli* JM109 was screened by restriction digests and qualitative enzyme assays and then, alkaline lysis miniprep plasmid DNA's of the correct constructs were electroporated into *P. fluorescens* MB214.

### Host Strain Pseudomonas fluorescens MB214

MB214 is a derivative of MB 101 (a wild-type prototrophic *P. fluorescens* strain), derived by a procedure wherein the *lacIZYA* operon (deleted of the *lacZ* promoter region) had been integrated into the chromosome to provide a host background where derivatives of the *lac* promoter can be regulated by lactose or IPTG. MB101 is Lac⁻ whereas MB214 is Lac⁺. However, MB101 can be rendered Lac+ by introducing an *E. coli lacI* gene on a plasmid into the strain.

### Example 1B. Expression of Extremophilic Cellulases

Seed cultures were produced as follows. *P. fluorescens* MB214 transformants were inoculated into 2-5 mL of Luria-Bertani Broth ("LB"), supplemented with 15µg/mL tetracycline HCl, in 15 ml Falcon tubes and growth for 16-20h, at 32°C, 300rpm. 1 mL of the seed culture (in LB) was placed into 50 mL of the Terrific Broth (TB) medium (see Table 4), supplemented with 15µg/mL tetracycline HCl, in 250ml bottom baffled shake-flasks, and incubated for 5h at 32°C, 300rpm. Induction was performed by the addition of IPTG to a final concentration of 0.5mM. Samples were taken at 16-24 hours post-induction.

| Table 4. TB Medium Recipe | |
|---|---|
| Bacto tryptone | 12 g/L |
| Bacto yeast extract | 24 |
| Glycerol | 10 |
| KH₂PO₄ | 2.3 |
| K₂HPO₄ | 12.5 |

Results for shake-flask scale results are presented in Table 5.

| Table 5. List of strains constructed and their performance in shake-flasks | | | |
|---|---|---|---|
| Expression Cassette | Strain | Isolates # | Cellulase Yield by SDS-PAGE (g//L) |
| P_{T5} | MB214pMYC1951 | 5 | 0.6 |
| | " " | 18 | 0.6 |
| P_{tac} | MB214pMYC1954 | 3.1 | 0.5 |
| | " " | 3.2 | 0.5 |
| | " " | 3.3 | 0.4 |
| | " " | 2.2 | 0.4 |
| | " " | 2.5 | 0.3 |

Results for 10-Liter scale results are presented in Table 6.

| Table 6. Performance of representative strains in 10 liter fermentations | | | |
|---|---|---|---|
| Expression Cassette | Strain | Isolates # | Cellulase Yield by SDS-PAGE (g/L) |
| P_{tac} | MB214pMYC1954 | 3.1 | 10 |
| | MB214pMYC1954 | 2.2 | 7 |
| | MB214pDOW2408 | - | 1.2 |

The cellulases were expressed at levels above 8% tcp in both shake-flask and high cell density fermentor cultures. The cellulases were separated and tested for activity and were found to be active.

### Example 2. Extremophilic Amylases

Alpha-amylase genes from a *Thermococcal* and a *Sulfolobus solfataricus* source were PCR amplified and cloned onto pMYC1803 as in Example 1, so that they became operably linked to a control sequence including the P_{tac} promoter in, an RSF1010-based vector also carrying a tetracycline resistance marker, as shown in Figure 1. The resulting constructs were transformed into LacI⁺ *P. fluorescens* MB101. The resulting recombinant host cells were cultured in 10 L fermentors by growth in a mineral salts medium (supplemented with tetracycline and fed with glucose or glycerol). The transformants were grown in fed-batch fermentation cultures, ultimately to cell densities providing biomasses within the range of about 20 g/L to more than 70 g/L (dry cell weight). The gratuitous inducer of the P_{tac} promoter, IPTG, was added to induce expression. Thereupon, the amylases were expressed (*i*.*e*. over-expressed) to a level within the range of about 5% tcp to more than 30% tcp. Thus, total productivity ranged from about 2 g/L to over 10 g/L, offering a yield above 100 g of extremozyme from a single 10 L batch. After host cell lysis, the extremozymes were purified by microfiltration followed by ultrafiltration. The resulting enzymes were characterized and further tested for starch liquefaction activity and found to be active, hyperthermophilic, and acidophilic.

### Example 3. Extremophilic Proteases

*Pyrococcus furiosus* and *Sulfolobus acidocaldarius* protease genes respectively encode pyrolysin (IUBMB EC 3.4.21.-), a serine protease active at 115°C and pH 6.5-10.5, and thermopsin (IUBMB EC 3.4.23.42), an acid protease operating optimally at 90°C and pH 2.0, respectively. These genes were PCR amplified and cloned onto pMYC1803 as in Example 1, so that they became operably linked to a control sequence including the P_{tac} promoter in an RSF1010-based vector also carrying a tetracycline resistance marker, as shown in Figure 1. The resulting constructs were transformed into LacI⁺ *P. fluorescens* MB214. The resulting recombinant host cells were cultured in 10 L fermentors by growth in a mineral salts medium (supplemented with tetracycline and fed with glucose or glycerol). The transformants were grown in fed-batch fermentation cultures, ultimately to cell densities providing biomasses within the range of about 20 g/L to more than 70 g/L (dry cell weight). Upon induction with IPTG, the proteases were expressed to levels within the range of about 5% tcp to more than 30% tcp. Thus, total productivity ranged from about 1 g/L to over 10 g/L, offering a yield above 100 g of extremozyme from a single 10 L batch.

### References

1. C. Nieto et al., Cloning vectors, derived from a naturally occurring plasmid of Pseudomonas savastanoi, specifically tailored for genetic manipulations in Pseudomonas, Gene 87:145-149 (1990).
2. M. Bagdasarian et al., Molecular and functional analysis of the broad host range plasmid RSF1010 and construction of vectors for gene cloning in Gram-negative bacteria, in Microbial Drug Resistance: Proceedings of the Third Int'l Symp., pp.183-97 (Tokyo, 1982).
3. J.P. Fuerste et al., Molecular cloning of the plasmid RP4 primase region in a multi-host-range tacP expression vector, Gene 48:119-131 (1986).
4. E. Brunschwig & A. Darzins, A two-component T7 system for the overexpression of genes in Pseudomonas aeruginosa, Gene 111:35-41 (1992).
5. R. Leemans et al., A broad-host-range expression vector based on the pL promoter of coliphage λ: regulated synthesis of human interleukin 2 in Erwinia and Serratia species, J. Bact. 169:1899-1904 (1987).
6. J.L. Ramos et al., Broad-host-range expression vectors containing manipulated meta-cleavage pathway regulatory elements of the TOL plasmid, FEBS Lett. 226:241-246 (1988).
7. N.T. Keen et al., Improved broad-host-range plasmids for DNA cloning in Gram-negative bacteria, Gene 70:191-197 (1988).
8. J.M. Blatny et al., Construction and use of a versatile set of broad-host-range cloning and expression vectors based on the RK2 replicon. Appl. Env. Microbiol. 63:370-379 (1997); and J.M. Blatney et al., Improved broad-host-range RK2 vectors for high and low regulated gene expression levels in Gram-negative bacteria, Plasmid 38:35-51 (1997).
9. A.A. Watson et al., Construction of improved vectors for protein production in Pseudomonas aeruginosa, Gene 172:163-164 (1996).
10. H.P. Schweizer et al., Vector design and host systems for Pseudomonas, in J.K. Setlow (ed.), Genetic Engineering, vol. 23 (2001) (Kluwer Plenum Press, New York).
11. J. Eichler, Biotechnological uses of archaeal extremozymes, Biotech. Adv. 19:261-78 (2001).
12. M.C. Srinivasan et al., Production and application of enzymes stable to and active under extreme environments: an overview, Proc. Indian Nat'l Sci. Acad. 65(Pt. B):143-62. (1999).
13. G.A. Sellek & J.B. Chaudhuri, Biocatalysis in organic media using enzymes from extremophiles, Enz. Microb. Technol. 25:471-82 (1999).
14. K.O. Stetter, Extremophiles and their adaptation to hot environments, FEBS Lett. 452:22-25 (1999).
15. M.W.W. Adams et al., Extremozymes: Expanding the limits of biocatalysis, Bio/technology 13:662-68. (1995).
16. D.C. Demirjian et al., Enzymes from extremophiles, Curr. Opin. Chem. Biol. 5:144-51 (2001).
17. E. Leveque et al., Thermophilic archaeal amylolytic enzymes, Enz. Microb. Technol. 26:3-14. (2000).
18. F. Niehaus et al., Extremophiles as a source of novel enzymes for industrial application, Appl. Microbiol. Biotech. 51:711-29 (1999).
19. C. Vieille & G.J. Zeikus, Hyperthermophilic enzymes: Sources, uses, and molecular mechanisms for thermostability, Microbiol. Mol. Biol. Rev. 65:1-43 (2001).
20. D.W. Hough & M.J. Danson, Extremozymes, Curr. Opin. Chem. Biol. 3:39-46 (1999).

## Claims

1. A recombinant cell containing an expression vector comprising a nucleic acid sequence encoding an exogenous extremozyme, wherein the recombinant cell is a Pseudomonad, and wherein the extremozyme is a hydrolase of class IUBMB EC 3 and exhibits an optimum of at least one catalytic property under at least one extremophilic condition selected from:
hyperthermophilic conditions of 70-130+°C; psychrophilic conditions of -2-20°C; halophilic conditions of 2-5M salt; acidophilic conditions of a pH of ≤ 4.5; alkalophilic conditions of a pH ≥ 9; piezophilic conditions of 10-80+ MPa; and xerophilic conditions of a_{w} < 0.85.

2. The recombinant cell of any preceding claim wherein the host cell is a Pseudomonas fluorescens cell.

3. The recombinant cell of any preceding claim wherein the extremozyme is selected from the group consisting of hyperthermophilic, psychrophilic, acidophilic, alkalophilic, and halophilic extremozymes.

4. A process for producing a recombinant cell according to any one of claims 1 to 3, the process comprising:
a) providing a host cell selected from a Pseudomonad; and
b) transforming the host cell with an expression vector comprising a nucleic acid including a coding sequence for an exogenous extremozyme, wherein the extremozyme is a hydrolase of class IUBMB EC 3 and exhibits an optimum of at least one catalytic property under at least one extremophilic condition selected from: hyperthermophilic conditions of 70-130+°C; psychrophilic conditions of -2-20°C; halophilic conditions of 2-5M salt; acidophilic conditions of a pH of ≤ 4.5; alkalophilic conditions of a pH ≥ 9; piezophilic conditions of 10-80+ MPa; and xerophilic conditions of a_{w} < 0.85.

5. A process for producing an extremozyme comprising growing a recombinant cell according to any one of claims 1 to 3 under conditions permitting expression of the extremozyme, wherein the extremozyme is expressed at an expression level of at least 5% total cell protein and is isolated.

6. The process of claim 5 wherein the cell is grown to a cell density of at least 40 grams per liter.

7. The process of claim 5 or 6 wherein the cell is grown in a fermentation scale of at least 10 liters.

8. The process of any one of claims 5 to 7 wherein the cell is grown in a mineral salts medium.

9. The process of any one of claims 5 to 8 wherein the extremozyme is purified.

10. The process of any one of claims 5 to 9 wherein the extremozyme undergoes a refolding step.

## Patentansprüche

1. Rekombinante Zelle, die einen Expressionsvektor enthält, umfassend eine Nukleinsäure-Sequenz, welche ein exogenes Extremozym kodiert, wobei die rekombinante Zelle ein Pseudomonad ist, und wobei das Extremozym eine Hydrolase der Klasse IUBMB EC 3 ist und ein Optimum von mindestens einer katalytischen Eigenschaft unter mindestens einer extremophilen Bedingung aufweist, die ausgewählt ist aus:
hyperthermophilen Bedingungen von 70-130+°C; psychrophilen Bedingungen von -2-20°C; halophilen Bedingungen von 2-5 M Salz; acidophilen Bedingungen bei einem pH-Wert von ≤ 4,5; alkalophilen Bedingungen bei einem pH-Wert von ≥ 9; piezophilen Bedingungen von 10-80+ MPa; und xerophilen Bedingungen von a_{w} < 0,85.

2. Rekombinante Zelle nach einem der vorhergehenden Ansprüche, wobei die Wirtszelle eine Pseudomonas fluorescens-Zelle ist.

3. Rekombinante Zelle nach einem der vorhergehenden Ansprüche, wobei das Extremozym aus der Gruppe bestehend aus hyperthermophilen, psychrophilen, acidophilen, alkalophilen und halophilen Extremozymen ausgewählt ist.

4. Verfahren zur Herstellung einer rekombinanten Zelle nach einem der Ansprüche 1 bis 3, wobei das Verfahren umfasst:
a) Bereitstellen einer Wirtszelle, die aus einem Pseudomonaden ausgewählt ist; und
b) Transformieren der Wirtszelle mit einem Expressionsvektor, der eine Nukleinsäure-Sequenz umfasst, welche eine kodierende Sequenz für ein exogenes Extremozym umfasst, wobei das Extremozym eine Hydrolase der Klasse IUBMB EC 3 ist und ein Optimum von mindestens einer katalytischen Eigenschaft unter mindestens einer extremophilen Bedingung aufweist, die ausgewählt ist aus:
hyperthermophilen Bedingungen von 70-130+°C; psychrophilen Bedingungen von -2-20°C; halophilen Bedingungen von 2-5 M Salz; acidophilen Bedingungen bei einem pH-Wert von ≤ 4,5;
alkalophilen Bedingungen bei einem pH-Wert von ≥ 9;
piezophilen Bedingungen von 10-80+ MPa; und xerophilen Bedingungen von a_{w} < 0,85.

5. Verfahren zur Herstellung eines Extremozyms, umfassend das Kultivieren einer rekombinanten Zelle nach einem der Ansprüche 1 bis 3 unter Bedingungen, die die Expression des Extremozyms erlauben, wobei das Extremozym mit einem Expressionslevel von mindestens 5% des Gesamtzellproteins exprimiert wird und isoliert wird.

6. Verfahren nach Anspruch 5, wobei die Zelle bis zu einer Zelldichte von mindestens 40 Gramm pro Liter kultiviert wird.

7. Verfahren nach Anspruch 5 oder 6, wobei die Zelle in einem Fermentationsmaßstab von mindestens 10 Litern kultiviert wird.

8. Verfahren nach einem der Ansprüche 5 bis 7, wobei die Zelle in einem Mineralsalzmedium kultiviert wird.

9. Verfahren nach einem der Ansprüche 5 bis 8, wobei das Extremozym aufgereinigt wird.

10. Verfahren nach einem der Ansprüche 5 bis 9, wobei das Extremozym einen Rückfaltungsschritt durchmacht.

## Revendications

1. Cellule recombinante contenant un vecteur d'expression comprenant une séquence d'acide nucléique qui code une extrêmozyme exogène, dans laquelle la cellule recombinante est un Pseudomonad et l'extrêmozyme est une hydrolase de la classe IUBMB EC3 qui présente un optimum d'au moins une propriété catalytique dans au moins une condition extrêmophile choisie parmi :
les conditions hyperthermophiles de 70 à plus de 130°C ; les conditions psychrophiles de -2 à 20°C ; les conditions halophiles de 2 à 5 M en sel ; les conditions acidophiles d'un pH inférieur à 4,5 ; les conditions alcalophiles d'un pH supérieur à 9 ; les conditions piézophiles de 10 à plus de 80 MPa ; et les conditions xérophiles de a_{w} <0,85.

2. Cellule recombinante selon l'une quelconque des revendications précédentes, dans laquelle la cellule-hôte est une cellule de Pseudomonas fluorescense.

3. Cellule recombinante selon l'une quelconque des revendications précédentes, dans laquelle l'extrêmozyme est choisie dans le groupe constitué des extrêmozymes hyperthermophile, psychrophile, acidophile, alcalophile, et halophile.

4. Procédé de production d'une cellule recombinante selon l'une quelconque des revendications 1 à 3, comprenant les étapes de :
a) fournir une cellule-hôte choisie à partir d'un Pseudomonad ; et
b) transformer la cellule-hôte avec un vecteur d'expression comprenant un acide nucléique qui inclut une séquence codant une extrêmozyme exogène, dans lequel l'extrêmozyme est une hydrolase de la classe IUBMB EC3 qui présente un optimum pour au moins une propriété catalytique dans au moins une condition extrêmophile choisie parmi : les conditions hyperthermophiles de 70 à plus de 130°C, les conditions psychrophiles de -2 à 20°C, les conditions halophiles de 2 à 5 M en sel, les conditions acidophiles d'un pH inférieur à 4,5 ; les conditions alcalophiles d'un pH supérieur à 9, les conditions piézophiles de 10 à plus de 80 MPa, et les conditions xérophiles de a_{w} <0,85.

5. Procédé de production d'une extrêmozyme comprenant le fait de cultiver une cellule recombinante selon l'une quelconque des revendications 1 à 3 dans des conditions qui permettent l'expression de l'extrêmozyme, l'extrêmozyme étant exprimée à un taux d'expression d'au moins 5% des protéines totales de la cellule et étant isolée.

6. Procédé selon la revendication 5, dans lequel la cellule est cultivée à une densité cellulaire d'au moins 40g/litre.

7. Procédé selon la revendication 5 ou 6, dans lequel la cellule est cultivée à une échelle de fermentation d'au moins 10 litres.

8. Procédé selon l'une quelconque des revendications 5 à 7, dans lequel la cellule est cultivée dans un milieu contenant des sels minéraux.

9. Procédé selon l'une quelconque des revendications 5 à 8, dans lequel l'extrêmozyme est purifiée.

10. Procédé selon l'une quelconque des revendications 5 à 9, dans lequel l'extrêmozyme subit une étape de repliement.
